(19) Europäisches Patentamt
European Patent Office
Office européen des brevets

(11) Veröffentlichungsnummer: **0 446 781 A1**

# (12) EUROPÄISCHE PATENTANMELDUNG

(21) Anmeldenummer: **91103473.4**

(51) Int. Cl.5: **C07C 209/90**, C07C 211/50

(22) Anmeldetag: **07.03.91**

(30) Priorität: **14.03.90 DE 4008074**

(43) Veröffentlichungstag der Anmeldung:
**18.09.91 Patentblatt 91/38**

(84) Benannte Vertragsstaaten:
**AT BE CH DE ES FR GB IT LI NL SE**

(71) Anmelder: **BASF Aktiengesellschaft**
**Carl-Bosch-Strasse 38**
**W-6700 Ludwigshafen(DE)**

(72) Erfinder: **Bruchmann, Bernd, Dr.**
**Giselherstrasse 79**
**W-6700 Ludwigshafen(DE)**
Erfinder: **Borschel, Eva-Marie, Dr.**
**Pfalzgrafenstrasse 53**
**W-6700 Ludwigshafen(DE)**
Erfinder: **Otto, Bernhard, Dr.**
**Buchenstrasse 12**
**W-6704 Mutterstadt(DE)**
Erfinder: **Langer, Werner, Dr.**
**Wittelsbachstrasse 41**
**W-6700 Ludwigshafen(DE)**
Erfinder: **Minges, Roland, Dr.**
**Auf er Setz 23**
**W-6718 Gruenstadt(DE)**
Erfinder: **Straehle, Wolfgang, Dr.**
**Hampeweg 9**
**W-6900 Heidelberg(DE)**
Erfinder: **Van Pee, Willy, Dr.**
**Bunderbeeklaan 18**
**B-2080 Kapellen(BE)**

## (54) Verfahren zur Qualitätsverbesserung von rohen Diaminodiphenylmethan.

(57) Verfahren zur Qualitätsverbesserung von rohen Diaminodiphenylmethanen, in dem man sie in Gegenwart eines Hydrierkatalysators mit Wasserstoff behandelt.

EP 0 446 781 A1

Die vorliegende Erfindung betrifft ein neues Verfahren zur Qualitätsverbesserung von rohen Diaminodiphenylmethanen.

Das großtechnisch durch sauer katalysierte Umsetzung von Anilin mit Formaldehyd erhaltene Gemisch verschiedener Kondensationsprodukte, der Diaminodiphenylmethane, üblicherweise auch als MDA bezeichnet, besteht zu überwiegenden Mengen aus dem zweikernigen p,p'-Isomeren

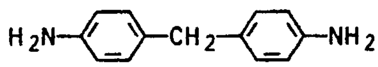

Man benötigt das MDA hauptsächlich zur Herstellung der entsprechenden Diphenylmethandiisocyanate ("MDI"), indem man es mit Phosgen umsetzt. Das MDI dient seinerseits als einer der wichtigsten Bausteine für die Herstellung von Polyurethanen.

Es ist allgemein bekannt, z.B. aus der DE-A 33 29 124 und der US-A 3 479 384, daß bei der Phosgenierung der MDA höchst unerwünschte Verfärbungen auftreten, die bei der Weiterverarbeitung des MDI zu den Polyurethanen erhalten bleiben.

Nach den genannten Patentveröffentlichungen unterwirft man das Roh-MDI daher bestimmten extraktiven Reinigungsverfahren. Diese Verfahren haben aber den Nachteil, daß die unerwünschten, die Verfärbung hervorrufenden Bestandteile nicht vermieden werden, sondern in einer zusätzlichen Aufarbeitungsstufe entfernt und entsorgt werden müssen.

Gemäß der US-A 4 465 639 erreicht man eine Aufhellung von MDI, indem man die Reaktionslösung direkt nach der Phosgenierung von MDA und vor dem Entfernen des überschüssigen Phosgens mit etwas Wasser behandelt. Eine zusätzliche Aufarbeitung wie bei den extraktiven Verfahren entfällt, jedoch führt der entstandene Chlorwasserstoff zu Korrosionsproblemen.

Der Erfindung lag daher die Aufgabe zugrunde, das Auftreten von Verfärbungen bei der Phosgenierung des MDA von vornherein zu vermeiden und somit die weitere aufwendige Reinigung des Roh-MDI entbehrlich zu machen.

Demgemäß wurde ein Verfahren zur Qualitätsverbesserung von rohen Diaminodiphenylmethanen gefunden, indem man sie in Gegenwart eines Hydrierkatalysators mit Wasserstoff behandelt.

Man kann das wäßrige Reaktionsgemisch, das bei der Umsetzung von Anilin und Formaldehyd zu MDA anfällt, unmittelbar der hydrierenden Behandlung unterwerfen und danach überschüssiges Anilin, Wasser und sonstige leicht siedende Komponenten abdestillieren, jedoch empfiehlt es sich, von einem bereits anilin- und wasserfreien Roh-MDA auszugehen. Dieses setzt sich in der Regel wie folgt zusammen:

50 - 95 Gew.-% 4,4'-Diaminodiphenylmethan
1 - 40 Gew.-% 2,4'-Diaminodiphenylmethan
0,1- 10 Gew.-% 2,2'-Diaminodiphenylmethan
10 - 40 Gew.-% drei- und vierkernige MDA-Homologe
1 - 10 Gew.-% höhermolekulare MDA-Homologe und unbekannte Substanzen.

Die Bedingungen der hydrierenden Behandlung sind nicht kritisch, wobei es sich jedoch von selbst versteht, solche scharfen Bedingungen zu vermeiden, unter denen die aromatischen Ringe angegriffen werden.

Im allgemeinen empfehlen sich ein Wasserstoffdruck von 1 bis 300 bar, bevorzugt von 10 bis 200 bar, besonders bevorzugt von 20 bis 60 bar und Temperaturen von 20 bis 400 °C, besonders von 70 bis 320 °c, so daß das Roh-MDA auch bei Normaldruck in flüssiger Phase vorliegt.

Als Hydrierkatalysatoren kommen sämtliche für die katalytische Hydrierung bekannten Katalysatoren in Betracht, sei es in Form von Substanz- oder Trägerkatalysatoren.

Als aktive Masse besonders gut geeignet sind die Platinmetalle, darunter vor allem Platin und Palladium. Daneben sind auch die üblichen Hydrierkatalysatoren wie Raney-Nickel, Metalle oder Oxide von Wolfram und Molybdän oder Gemische verschiedener Metalle, z.B. von Nickel und Molybdän, geeignet.

Geeignete Trägermaterialien sind beispielsweise Aluminiumoxid, Kieselgel, Silikate, Aktivkohle und Zeolithe.

Zweckmäßigerweise beträgt die Menge des Katalysators 0,001 bis 10 Gew.-% aktive Masse, bezogen auf die Menge des rohen MDA.

Das Verfahren kann kontinuierlich oder diskontinuierlich ausgeführt werden, wobei im diskontinuierlichen Betrieb die Zeitdauer, bis kein Wasserstoff mehr verbraucht wird, in der Regel zehn Minuten bis zwei Stunden beträgt.

Die Reaktion wird zweckmäßigerweise ohne Lösungsmittelzusatz durchgeführt. Jedoch können Lösungsmittel wie Kohlenwasserstoffe, z.B. n-Heptan und Cyclohexan, Alkohole, z.B. Methanol und Ethanol, und Ether, z.B. Tetrahydrofuran und 1,4-Dioxan, in Mengen von 0,05 bis 5 kg pro kg MDA mitverwendet werden. Besonders gute Ergebnisse erzielt man, wenn man die hydrierende Behandlung des Roh-MDA in Gegenwart von 0,5 bis 20 Gew.-% Wasser vornimmt.

Das aus hydrierend behandeltem MDA hergestellte MDI ist hellgelb bis dunkelgelb und eignet sich ohne weitere Reinigung für die Herstellung von Polyurethanen.

Beispiel 1

Diskontinuierliche hydrierende Behandlung von Roh-MDA

500 g von Wasser und überschüssigem Anilin befreites Roh-MDA, welches durch saure Kondensation von Anilin mit Formaldehyd hergestellt worden war und 55 Gew.-% 4,4'-Diaminodiphenylmethan, 3 Gew.-% 2,4'-Diaminodiphenylmethan, 1 Gew.-% 2,2'-Diaminodiphenylmethan, 25 Gew.-% drei- und 10 Gew.-% vierkernige MDA-Verbindungen und 6 Gew.-% höherkernige MDA-Verbindungen und unbekannte Substanzen enthielt, wurde bei 80 °C und 50 bar Wasserstoffdruck in Gegenwart von 5 g eines pulverförmigen Trägerkatalysators aus 5 Gew.-% Palladium und 95 Gew.-% Aluminiumoxid für 2 Stunden der hydrierenden Behandlung unterworfen, wonach der Katalysator abgetrennt wurde.

Beispiel 2

Diskontinuierliche Behandlung von Roh-MDA

Zu einem anilin- und wasserfreien MDA-Gemisch mit derselben Zusammensetzung wie in Beispiel 1 wurden 25 g Wasser zugesetzt. Diese Mischung wurde bei 100 °C und 50 bar Wasserstoffdruck in Gegenwart von 5 g eines pulverförmigen Trägerkatalysators aus 5 Gew.-% Palladium und 95 Gew.-% Aluminiumoxid für 2 Stunden der hydrierenden Behandlung unterworfen, wonach der Katalysator abgetrennt wurde.

Beispiel 3

Kontinuierliche hydrierende Behandlung von Roh-MDA

50 g/h des Roh-MDA gemäß Beispiel 1 wurden kontinuierlich über 50 g eines Festbettkatalysators aus 3 Gew.-% Palladium auf Aluminiumoxid geleitet und hierbei bei 150 °C und 50 bar Wasserstoffdruck der hydrierenden Behandlung unterworfen.

Beispiel 4

Kontinuierliche hydrierende Behandlung von Roh-MDA

50 g/h eines anilin- und wasserhaltigen MDA-Gemisches, das 42 Gew.-% 4,4'-Diaminodiphenylmethan, 2 Gew.-% 2,4'-Diaminodiphenylmethan, 0,5 Gew.-% 2,2'-Diaminodiphenylmethan, 19 Gew.-% drei- und 8 Gew.-% vierkernige MDA-Verbindungen, 5 Gew.-% Wasser, 20 Gew.-% Anilin und 3,5 Gew.-% höherkernige MDA-Verbindungen und unbekannte Substanzen enthielt, wurden kontinuierlich über 50 g eines Festbettkatalysators aus 5 Gew.-% Nickel und 12,7 Gew.-% Molybdän auf Aluminiumoxid geleitet und hierbei bei 300 °C und 30 bar Wasserstoffdruck der hydrierenden Behandlung unterworfen.

Beispiel 5

Qualitätsprüfung des nach den Beispielen 1 bis 4 erhaltenen Rein-MDA

Das nach den Beispielen 1 bis 4 erhaltene MDA wurde destillativ von etwaig vorhandenem Wasser und/oder Anilin befreit und in mit Phosgen gesättigtem Chlorbenzol bei einem Phosgendruck von 2 bar zum MDI phosgeniert. Proben des MDI wurden mit dem 5-fachen Volumen Chlorbenzol verdünnt, wonach die Farbzahlen dieser Lösungen nach DIN 6162 ermittelt wurden.

Zum Vergleich wurde die Farbzahl des MDI ermittelt, welches aus anilin-und wasserfreiem Roh-MDA ohne hydrierende Behandlung hergestellt worden war. Die Ergebnisse dieser Versuche sind der nachste-

henden übersicht zu entnehmen.

| MDI, hergestellt unter Verwendung von MDA | Farbe (visuell) | Farbzahl (nach DIN 6162) |
|---|---|---|
| <u>erfindungsgemäß</u> | | |
| Beispiel 1 | hellgelb | 40 |
| Beispiel 2 | hellgelb | 20 |
| Beispiel 3 | hellgelb | 20 |
| Beispiel 4 | hellgelb | 50 |
| <u>zum Vergleich</u> | | |
| ohne hydrierende Vorbehandlung | dunkelbraun | 180 |

**Patentansprüche**

1. Verfahren zur Qualitätsverbesserung von rohen Diaminodiphenylmethanen, dadurch gekennzeichnet, daß man sie in Gegenwart eines Hydrierkatalysators mit Wasserstoff behandelt.

2. Verfahren nach Anspruch 1, dadurch gekennzeichnet, daß der Wasserstoffdruck hierbei 1 bis 300 bar beträgt.

3. Verfahren nach den Ansprüchen 1 oder 2, dadurch gekennzeichnet, daß man es bei 20 bis 400 °C vornimmt.

4. verfahren nach den Ansprüchen 1, 2 oder 3, dadurch gekennzeichnet, daß man es in Gegenwart von 0,5 bis 20 Gew.-% Wasser, bezogen auf das Roh-MDA, durchführt.

Europäisches Patentamt

# EUROPÄISCHER RECHERCHENBERICHT

Nummer der Anmeldung

**EP 91 10 3473**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 262 562 (AIR PRODUCTS & CHEMICALS INC.)<br>* Seite 11, Run 4 * | 1 | C 07 C 209/90<br>C 07 C 211/50 |
| A | US-A-3 068 289 (J.E. WOODBRIDGE)<br>* Insgesamt * | 1 | |
| A | US-A-3 154 583 (B.A. DOMBROW)<br>* Ingsesamt * | 1 | |
| A | EP-A-0 231 788 (AIR PRODUCTS & CHEMICALS INC.)<br>* Insgesamt * | 1 | |
| A | EP-A-0 105 146 (BAYER AG)<br>* Beispiel 5 * | 1 | |

RECHERCHIERTE SACHGEBIETE (Int. Cl.5)

C 07 C 209/00
C 07 C 211/00

Der vorliegende Recherchenbericht wurde für alle Patentansprüche erstellt

| Recherchenort | Abschlußdatum der Recherche | Prüfer |
|---|---|---|
| Den Haag | 12 Juni 91 | WELLS A.G. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument